(19) Europäisches Patentamt
European Patent Office
Office européen des brevets

(11) **EP 2 962 233 B1**

(12) **EUROPEAN PATENT SPECIFICATION**

(45) Date of publication and mention
of the grant of the patent:
**21.11.2018 Bulletin 2018/47**

(51) Int Cl.:
***G16H 50/20*** (2018.01)

(21) Application number: **14709900.6**

(22) Date of filing: **28.02.2014**

(86) International application number:
**PCT/EP2014/053890**

(87) International publication number:
**WO 2014/131863 (04.09.2014 Gazette 2014/36)**

(54) **METHOD FOR ASSESSING ACTIVITY OF AN AUTOINFLAMMATORY DISEASE**

VERFAHREN ZUR BEURTEILUNG DER AKTIVITÄT EINER AUTOINFLAMMATORISCHEN ERKRANKUNG

PROCÉDÉ D'ÉVALUATION DE L'ACTIVITÉ D'UNE MALADIE AUTO-INFLAMMATOIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.02.2013 EP 13305231**

(43) Date of publication of application:
**06.01.2016 Bulletin 2016/01**

(73) Proprietors:
• **Assistance Publique Hôpitaux De Paris
75004 Paris 4 (FR)**
• **Istituto G. Gaslini
16147 Genova (IT)**
• **UNIVERSITE PARIS-SUD (PARIS XI)
91470 Orsay Cedex (FR)**

(72) Inventors:
• **KONÉ-PAUT, Isabelle
F-75016 Paris (FR)**
• **PIRAM, Maryam
F-94240 L'Hay Les Roses (FR)**
• **GATTORNO, Marco
I-16145 Genova (IT)**
• **RUPERTO, Nicolino
I-16035 Rapallo (IT)**

(74) Representative: **Flesselles, Bruno F.G.
BF IP
36 rue Jean de la Fontaine
75016 Paris (FR)**

(56) References cited:
**US-A1- 2006 135 859**

• **M. PIRAM ET AL: "A preliminary score for the assessment of disease activity in hereditary recurrent fevers: results from the AIDAI (Auto-Inflammatory Diseases Activity Index) Consensus Conference", ANNALS OF THE RHEUMATIC DISEASES - AUTHOR MANUSCRIPT, vol. 70, no. 2, 15 November 2011 (2011-11-15), pages 309-314, XP055074583, ISSN: 0003-4967, DOI: 10.1136/ard.2010.132613 cited in the application**
• **F. YALCINKAYA ET AL: "A new set of criteria for the diagnosis of familial Mediterranean fever in childhood", RHEUMATOLOGY, vol. 48, no. 4, April 2009 (2009-04), pages 395-398, XP055074614, ISSN: 1462-0324, DOI: 10.1093/rheumatology/ken509**
• **M. GATTORNO ET AL: "A diagnostic score for molecular analysis of hereditary autoinflammatory syndromes with periodic fever in children", ARTHRITIS & RHEUMATISM, vol. 58, no. 6, June 2008 (2008-06), pages 1823-1832, XP055074722, ISSN: 0004-3591, DOI: 10.1002/art.23474**

**Description**

The invention provides a method and device making it possible to identify the activity of an autoinflammatory disease in a patient.

The hereditary recurrent fever syndromes (HRFs) are rare Mendelian autoinflammatory diseases (AID) characterized by flares of fevers associated with acute inflammation affecting various tissues without evidence of an underlying cause. With the exception of familial Mediterranean fever (FMF) these diseases are very rare with estimated prevalence of less than two per million.

These autoinflammatory diseases (AIDs) are caused by primary dysfunction of the innate immune system. Proteins that are mutated in AIDs mediate the regulation of NFkappaB activation, cell apoptosis, and IL-1beta secretion through cross-regulated and sometimes common signaling pathways. These conditions are characterized by recurrent attacks of fever, abdominal pain, arthritis, and cutaneous signs; these symptoms sometimes overlap, obscuring diagnosis. The

The four main diseases by date of description and frequency are: FMF, mevalonate kinase deficiency (MKD), tumor necrosis factor receptor (TNF)-associated periodic syndrome (TRAPS), and cryopyrin-associated periodic syndromes (CAPS). Recent advances in the molecular pathogenesis of HRFs have led to a better understanding of the common pathways and mediators of apoptosis, inflammation and cytokine signaling involved in their pathogenesis and have radically improved diagnosis and therapies (Touitou et Kone-Paut, Autoinflammatory diseases. Best Pract Res Clin Rheumatol 2008;22(5):811-29; Hashkes et al., Ann Intern Med 2012;157(8):533-41)..

With the increasing potential for targeted therapies in AID, there is the need for validated assessment tools which can be used to evaluate the level of disease activity and response to therapy and thus to assess drug efficacy in standardize assessments across trials (Singh et al., Arthritis Rheum 2006;55(3):348-52; Hoffman et al., Arthritis Rheum 2008;58(8):2443-52; Lachmann et al., N Engl J Med 2009;360(23):2416-25; Gillespie et al., J Inflamm Res 2010;3:1-8)

The lack of such standardized and validated measures for assessing disease activity for either adults or children with AID, has seriously hampered assessment of current treatments and comparison of treatment responses in the different HRFs.

It is therefore important to design a method that would help the physician to determine whether the AID is active in the patient or whether it is controlled by the drugs that are being taken.

An international collaboration, initiated by Assistance Publique-Hôpitaux de Paris (APHP) in association with the Paediatric Rheumatology International Trials Organization (PRINTO at www.printo.it) and supported by the EUROFEVER and EUROTRAPS networks has already designed the content and preliminary scoring of this Auto-Inflammatory Disease Activity Index (AIDAI) using a single format disease adapted patient diary for the four major HRFs (Piram et al., Ann Rheum Dis 2011;70(2):309-14).

Piram *et al.* (*op. cit*.) thus described the design of various Indexes that are each specific for one of the four main diseases as indicated above. For each index, the patients had to daily give a note for some items associated to their disease (See Table 1). Fever was noted as 0 (absent) or 1 (present) whereas the other disease-specific variables were to be scored from 0 to 3 according to their severity (0=absent, 1=minor, 2=mild, 3=severe).

Table 1: Variables selected in Piram *et al.* (*op. cit.*) to assess disease activity

| FMF | MKD | TRAPS | CAPS |
|---|---|---|---|
| Fever ≥38°C | Fever ≥38°C | Fever ≥38°C | Fever ≥38°C |
| Abdominal pain | Abdominal pain | Abdominal pain | Limb pain |
| Arthralgia or myalgia | Nausea/vomiting | Limb pain | Conjunctivitis |
| Swelling of the joints | Diarrhea | Eye manifestations | Headaches |
| Chest pain | Limb pain | Skin rash | Skin rash |
| Skin rash | Painful lymph nodes | Overall TRAPS symptoms | |
| CAPS, cryopyrin-associated periodic syndromes; FMF, familial Mediterranean fever; MKD, mevalonate kinase deficiency; TRAPS, TNF receptor-1-associated periodic syndrome. | | | |

A final score was generated by calculating the sum of the scores for all variables divided by the number of days over which the diary was completed. Scores could vary from 0 to 16 (0-13 in CAPS). Piram et al clearly indicated that the scoring system is a preliminary system which measures disease activity for each of the four hereditary autoinflammatory disorders. Furthermore, a validation phase was to be made (in order to determine the threshold above which each disease is considered to be active).

The purpose of the present invention is to provide a validated score that could be generic to detect activity for all AIDs, and would be easier to obtain. Indeed, it may be difficult for the patient to grade the different variables associated to the disease from 0 to 3, in particular for children to discriminate between a mild and a severe item. This score has been validated through expertise of the physicians having assessed the disease activity of the patients.

A new score and method for detecting activity of an AID is thus provided, where some items are scored every day by the patient and a total score is then obtained. Depending on the amount of this score, said AID is then considered as active or not. This test and method is thus useful, in particular, to adapt the patient's treatment if it proves not effective enough.

In particular, the new AIDAI score uses a simplified scoring system. The patient is requested to score multiple items associated to the AID disease, but each item is dichotomized (i.e. is scored 0 (absence of the sign) or 1 (presence of the sign)) rather than from 0 to 3 as in the previously described index. Surprisingly, reducing the granularity of the scoring didn't lower the performance of the test, as determined by statistical analysis, while allowing for greater simplicity in the AIDAI completion.

The invention thus relates to a computer-implemented method for determining the activity of an auto-inflammatory disease in a patient, comprising the steps of:

a) Having said patient daily report, during a one-month period, the status of 12 dichotomous items (as disclosed below), wherein each item corresponds to a physiological observation

b) Recovering said reports from said patient

c) Allocating the value "1" to each item which has received a positive completion by said patient (presence of the item) and the value "0" to each item which has received a negative completion by said patient (absence of the item)

d) Combining, in a mathematical function as disclosed below, all the values obtained in step c) for each days of a consecutive period of one month in order to obtain an end value wherein said auto-inflammatory disease is active in said patient if said end-value is higher than a predetermined cut-off. Said predetermined cut-off is 9.

This method is computer-implemented and preferably performed by the physician/clinician. The method may also comprise the step of analyzing said end value of said mathematical function in order to determine the presence of active AID in said patient. In particular, it is possible to determine that said auto-inflammatory disease is active in said patient if said end-value is higher than a predetermined cut-off of value 9.

Said auto-inflammatory disease is a major hereditary recurrent fever syndrome and is FMF, mevalonate kinase deficiency (MKD), tumor necrosis factor receptor (TNF)-associated periodic syndrome (TRAPS), or cryopyrin-associated periodic syndromes (CAPS).

Also herein described is a method for determining the activity of an auto-inflammatory disease in a patient, comprising the steps of:

a) Providing a one-month diary to said patient, wherein said diary comprises an emplacement for each day of the month and wherein each daily emplacement of said diary comprises a [the same] list of 12 dichotomous items (as disclosed below), wherein each item corresponds to a physiological observation

b) Recovering said diary from said patient, wherein said diary has been completed daily by said patient for a consecutive period of one month

c) Allocating the value "1" to each item which has received a positive completion by said patient (presence of the item) and the value "0" to each item which has received a negative completion by said patient (absence of the item)

d) Combining all values obtained in step c) in a mathematical function in order to obtain an end value

e) Determining that said auto-inflammatory disease is active in said patient if said end-value is higher than a predetermined cut-off, wherein said auto-inflammatory disease is a major hereditary recurrent fever syndrome.

Said cut-off value is 9.

As used herein, an autoinflammatory disease is intended to mean a disease in which the innate immune system causes inflammation. These diseases are relatively new diseases, which are characterized by intense episodes of inflammation that result in such symptoms as fever, rash, or joint swelling.

One can cite major hereditary recurrent fever syndromes (FMF, TRAPS, MKD, CAPS) as described above, as well as Neonatal Onset Multisystem Inflammatory Disease (NOMID or CINCA), Deficiency of the Interleukin-1 Receptor Antagonist (DIRA) or Behçet's Disease. Autoinflammatory diseases also encompass Blau, Majeed, and PFAPA syndromes. A review of these diseases can be found in Grateau (Acta Clin Belg. 2006 Sep-Oct; 61(5):264-9).

In the context of the invention, the autoinflammatory disease is chosen among FMF, mevalonate kinase deficiency (MKD), tumor necrosis factor receptor (TNF)-associated periodic syndrome (TRAPS), and cryopyrin-associated periodic syndromes (CAPS).

By "daily reporting the status of a dichotomous item, wherein said item corresponds to a physiological observation", one intends to indicate that the patient will note whether or not said physiological condition has been present or absent on that specific day of report. This can be performed, for instance, by checking a box corresponding to both said item and said specific day. Such box crossing would then amount to a positive completion.

Step b) of the method consists in recovering the daily reports from said patient. In a specific embodiment, said recovery is made directly by said patient to said physician/clinician.

In another embodiment, step a) is performed by the patient filling out an electronic form, in particular on a patient's device, and the recovery of step b) consists in sending the filled form to a server that can be consulted by the physician/clinician. In this embodiment, an "electronic form" relates to a form that can be completed electronically by the patient (form user) and for which the data is transmitted into a database or another application after completion.

Said electronic form can be accessed through a dedicated website to which the patient connects, preferably with an identifier and password. Step b) (transmission of the data) is then performed when the patient validates the form. In this embodiment, one can design the website such as to provide a blank electronic form to said patient each day, until it is validated. In this embodiment, it is possible and known in the art to automatically remind the patient to fill the electronic form every day (such as by sending a daily automatic email, or a Short Message on the device of the patient (SMS)). It is also possible to send such reminders when the patient has not logged in or validated the form for one day. The server at the receiving end can perform a daily check for completion of the form for the patient and send a reminder in case the form has not been completed during a specified period of time.

In another embodiment, said electronic form is presented to the patient on a phone (smartphone) through a specifically designed application present on said phone. Said application may also contain other features such as opening a pop-up window every day or being linked to the calendar of said phone's user in order to remind the patient to fill-out the form. The application may also present the items one at a time to the user (one item is presented after the previous one has been completed) and send the data once (perform step b)) after the last item has been completed, and the patient has validated the data entry, and when network (such as wireless network, GSM, 3G or wifi) is available.

When an electronic form is used, said entered data is sent to a distant server, according to method known in the art. It is preferred when communication between the distant server and the patient's device (either computer or phone) is encrypted.

"Combining, in a mathematical function, all the values obtained in step c)for each days of a consecutive period of one month in order to obtain an end value" is intended to mean that, after all values (1 or 0) have been generated for all items for all days of the one-month period, these values are then introduced in a mathematical function. In a preferred embodiment, said mathematical function is the algebraic sum of the values obtained in c). In this embodiment, all values are given the same weight in said function.

Although not claimed in the context of the invention, one or more values are given a greater or lesser weight in the mathematical function. One could use some coefficient between some values linked to a particular item (for instance, fever could be given a higher (or lower) weight, which would be translated by allocating a coefficient higher than 1 (or lower than 1) for all values obtained for the fever item before adding these values to the other ones). In this embodiment, the modified weight given to some values is not necessarily identical (i.e. some values may be given more weight than others, or some values may be given an increased weight, whereas other values may be given a decreased weight). Statistical analysis could help determine the proper weight for each value.

In the context of the invention, though, all values are given the same weight (i.e. the mathematical function is the algebraic sum of all values).

When the reporting of step b) is performed through electronic/network means (either when the patient uses a computer or a smartphone to enter the data), the data is then received on a remote server (remote from the source). Allocation of the 0/1 value of step c) and calculation of the end value in step d) can thus be performed automatically, and said end value (calculated daily on a one month rolling) can be sent to the physician (by any method known in the art such as by email). The physician can thus determine daily the status of the activity of the disease for said patient.

Said items reported by the patient consist of a) Fever $\geq$38°C, b) Overall symptoms of the disease, c) Abdominal pain, d) Nausea/vomiting, e) Diarrhea, f) Headaches, g) Chest pain, h) Painful nodes, i) Arthralgia or Myalgia, j) Swelling of the joints, k) Eyes manifestations, l) Skin rash.

Overall symptoms of the disease are symptoms that are not listed apart in the group specified above. In these kinds of diseases, the patient can recognize a burst of the disease (such as an increased fatigue, irritability and the like). The patient can thus indicate whether he/she feels that there was such a disease burst on that specific day (this is particularly true for TRAPS).

Although not claimed, the application describes that said patient can daily report, during a one-month period, the status of exactly 5 dichotomous items.

In this case, said group of five items shall preferably be (fever $\geq$38°C / headaches / arthralgia or myalgia / eyes manifestations / skin rash).

Although not claimed, the application describes that said patient can daily report, during a one-month period, the status of exactly 6 dichotomous items.

In this embodiment, said group of five items shall preferably be chosen in the group consisting of (fever ≥38°C / abdominal pain / chest pain / arthralgia or myalgia / swelling of the joints / skin rash), (fever ≥38°C / abdominal pain / nausea-vomiting / diarrhea / painful nodes / arthralgia or myalgia) and (fever ≥38°C / overall symptoms / abdominal pain / arthralgia or myalgia / eyes manifestations / skin rash).

Although not claimed, the application describes that said patient can daily report, during a one-month period, the status of exactly 7, 8, 9, 10 or 11 items.

In the context of the invention, said patient shall daily report, during a one-month period, the status of exactly the twelve dichotomous items as indicated above as a) to l).

In another embodiment, said patient may also report other information, such as the intake of pain killers, the number of days out of school or work, any disturbance of the social life and the like.

The patient may also report any information related to an item, which may also help to discard the report of said item on a particular day, or a period of time. For instance, in case the patient has had the flu, the fever item during this period may be discarded. Any nausea/vomiting episodes may also be discarded if the patient has had gastrointestinal disease. Generally, the patient can recognize when a symptom is linked to activity of the disease or when it is linked to another disease. Preferably, the patient shall thus report only the symptoms linked to the AID. In practice, and in order to be sure not to miss any symptom, the patient is requested to report all information, and indicate, in an open box, any information that would help to discard or not the reported symptom (such as presence of another punctual disease).

In the context of the invention, said auto-inflammatory disease is a hereditary recurrent fever syndrome (HRFs), in particular chosen in the group consisting of familial Mediterranean fever (FMF), mevalonate kinase deficiency (MKD), tumor necrosis factor receptor-associated periodic syndrome (TRAPS), and cryopyrin-associated periodic syndromes (CAPS).

In the implemented method, said mathematical function is the algebraic sum of the values of step c) and the predetermined cut-off is 9. This means that when the sum of said values of step c) is above or equal to 9, said AID is active; when the sum of said values of step c) is lower than 9, said AID is not active.

The disclosure also describes a form to be filled by a patient, which comprises a list of the 12 dichotomous items disclosed above, wherein each item corresponds to a physiological condition that can be experienced by said patient, and a box to be checked by said patient for each item when said patient experiences said physiological condition.

In a specific embodiment, said form is an electronic form. In another embodiment, said form is a physical form, such as a paper sheet on which are printed the elements as mentioned above.

In another embodiment, said form also presents a list of days and wherein each of said 12 dichotomous items is to be reported on each day. This describes in particular a diary that would be given to a patient for filling for a one-month period, wherein said patient would have a box (to check or not) for each item for each day of this one-month period.

Although not claimed, the application describes when said form comprises five items. In another embodiment, said form comprises six items. Although not claimed, the application describes when said form comprises exactly 7, 8, 9, 10 or 11 items. In the context of the invention, said form contains 12 items.

As indicated above, said items consist of a) Fever ≥ 38°C, b) Overall symptoms of the disease, c) Abdominal pain, d) Nausea/vomiting, e) Diarrhea, f) Headaches, g) Chest pain, h) Painful nodes, i) Arthralgia or Myalgia, j) Swelling of the joints, k) Eyes manifestations, l) Skin rash.

In an embodiment, said form comprises an open box that can be filled by the patient with free text in order to report any element of interest for the physician/clinician.

**Description of the Figures**

Figure 1: Table 3: Scores of the AIDAI tool with items dichotomized as 0/1 applied in 98 patients with a HRF diagnosis. Data refer to a diary completed by the patients/parents in the month preceding the visit. Data are means ± standard deviation. FMF: familial Mediterranean fever; MKD: mevalonate kinase deficiency; TRAPS: tumor necrosis factor receptor-associated periodic syndrome; CAPS; and cryopyrin-associated periodic syndromes

Figure 2: Distribution of the AIDAI total score for 98 patients with a HRF diagnosis (0/1 scoring).

Figure 3: ROC curve with binary items of disease activity (0/1 scoring) showing an area under curve of 0.98(95% CI 0.96-1) in 98 patients with an HRF

**Examples**

Patients and Methods

The overall methodology of this project phase was based on the following framework:

Patients were asked to fill a survey (diary) for at least one month prior to assessment.

In a first step, physicians made a blinded evaluation of patient's disease activity level, through analysis of the results of the survey

For patients for which consensus was not reached, a NGT consensus conference was conducted, leading to assessment of the disease activity level for some of these patients.

Finally, the score was finalized, and the threshold above which activity is present was determined. Sensitivity and specificity could then be calculated.

This process was followed as there is no golden standard to determine the activity of the diseases, and the state of activity of the patients thus needs to be determined by the experience of various physicians, specialists of these diseases.

*Study design.*

A convenience sample of patients (children and adults) with a genetically confirmed diagnosis of FMF, MKD, TRAPS or CAPS, and in varying states of disease activity, ranging from active to inactive disease, was enrolled consecutively.

Patients were evaluated by at least one physician with expertise in the care of AID. All patients (or their parents for minors as appropriate) were asked to complete a one month prospective diary prior to the scheduled clinical appointment.

*Content and scoring of the AIDAI tool.*

The AIDAI diary contains 13 items as follows: a) fever $\geq$38°C (100.4°F); b) overall symptoms; c) abdominal pain; d) nausea/vomiting; e) diarrhea; f) headaches; g) chest pain; h) painful nodes; i) arthralgia or myalgia; j) swelling of the joints; k) eyes manifestations; l) skin rash; m) pain relief taken.

The items of patient/parent's diary were dichotomized as no (0) =absence of symptom or yes (1) =presence of symptom (either minor, mild or severe), yielding a total score in a single day of 0-12. In a month of 31 days the cumulative score thus ranges from 0 to 372.

Indeed, although the diary also noted the use of rescue (pain killers) treatment, this item was not used in the score calculation.

In addition to the diary completed by the patient and/or parents, physicians and patients completed together, during the visit, a questionnaire retrospectively assessing disease activity during the preceding 30 days: this documented: 1) the numbers of days with each symptoms; 2) all the treatments taken 3) the number of days of pain relief drugs; 4) the regularity of disease modifying anti-rheumatic drugs (DMARDs) taken (colchicine or biologics); 5) the number of days out of school or work; 6) patient's social life disturbance by the disease with a three level categorical scale (none; a little; a lot); 7) patient's assessment of fatigue on a 21 circle visual analogue scale (VAS) (where 0= no fatigue and 100=maximum fatigue); 8) patient's global assessment of overall well-being and physician's global assessment of disease activity on two separate 21 circle VAS (Filocamo et al., J Rheumatol 2010;37(7):1534-41; Pincus et al., J Rheumatol 2008;35(8):1550-8.); 9) the need to consult an external doctor; 10) the feasibility of the activity score (patient's opinion and comments).

*Validation procedures*

Data from patients/parents and physicians' assessment were validated through a three step validation process:

Step 1 Physician's blinded web evaluation of patient's disease activity level:

Seven physicians with expertise in AID evaluated the level of activity for each patient in the database as: no activity (0), low activity (1), moderate activity (2), severe activity (3). These physicians had access to the completed diary as well as to the questionnaire filled by the patients.

Patients were anonymized, total AIDAI score was not provided and the physicians were blinded to the diagnosis so that they could not recognize the patients followed in their own hospital.

The physicians worked independently from each other. At each session a minimum consensus of 6/7 (85%) for the inactive/active (low, moderate, and severe activity combined) was required to consider a patient as having reached a final consensus. When consensus was not achieved a new session was held for a total of three iterative independent evaluation sessions; at each subsequent session physicians were provided with their previous score as well as with the blinded evaluation of the other participants.

<u>Step 2 NGT consensus conference of patient's disease activity level:</u>

Eight physicians were asked to re-evaluate individually the level of disease activity for the patients in whom consensus was not achieved in the previous step. For all patients, each physician was asked to provide a verbal explanation as to why he/she has given a certain level of disease activity to that particular patient. The individual evaluation of each physician was shown on a screen to all participants. Then a web-based electronic vote was taken and consensus was achieved if a minimum of 6/8 (75%) participants provided the same level of disease activity. Patients for which consensus could not be achieved after a second vote were discarded from further considerations.

<u>Step 3 Statistical analysis of the AIDAI scoring system:</u>

In the work previously published (Piram *et al.*, *op. cit.*), the score calculation differs according to the specific disease as follows:

FMF could be scored by adding the variables a+c+g+i+j+l,
MKD the variables a+c+d+e+h+i,
TRAPS the variables a+b+c+i+k+l and
CAPS the variables a+f+i+k+l.

For a 31 day month, the cumulative scores of the prior art ranged from 0 to 496 for FMF, MKD and TRAPs and 0 to 403 for CAPS, respectively.
In the context of the work hereby carried out, it was determined that the sum of the positive items from the diary as yes/no has the same statistical performance of the disease specific scoring system previously described.
Furthermore, it was determined that it was possible to calculate a simplified score for each subject by summing up all the items of the diary as total score=a+b+c+d+e+f+g+h+i+j+k+l for the simplified items scored 0 to 1 (range 0 to 372).
This was also compared to the total score=a+b+c+d+e+f+g+h+i+j+k+l obtained for the items scored 0 to 3 (range 0 to 1054) in the previous test.
Average values of the score were compared between activity groups (inactive versus the three levels of activity combined). The ability of the score to discriminate active versus inactive patients and the best AIDAI total cut-off score to classify the patients as active/inactive was evaluated by a receiver operating characteristic (ROC) analysis. Sensitivity, specificity and accuracy of the scores were calculated using the best cut-off value of the total AIDAI score.
Data were entered in a web based Access XP database and analyzed with Excel XP (Microsoft), SPSS Inc. v.18 by 2 of the authors (MPS and NR).
It is reminded that:
Sensibility is the probability of obtaining a positive result for the test if the disease is active; Sensitivity = (TP) / (TP + FN). A test that is 100 % sensible has thus no false negative.
Specificity is the probability of obtaining a negative result for the test if the disease is not active; Specificity = (TN) / (TN + FP). A test that is 100 % specific has thus no false positive.

$$\text{Accuracy} = (TP + TN) / (TP + TN + FP + FN)$$

TP = True Positive (patients with active disease and above or equal to the threshold)
TN = True Negative (patients with disease not active and below the threshold)
FP = False Positive (patients with disease not active and above or equal to the threshold)
FN = False Negative (patients with active disease and below the threshold)

It is always important to have a test that is both specific and sensitive.
The quality of a test may be determined by drawing a Receiving Operating Characteristic (ROC) curve and measuring the Area Under Receiving Operating Characteristic curve (AUROC).
The ROC curve is drawn by plotting the sensitivity versus (1-specificity), after classification of the patients, according to the result obtained for the test, for different thresholds (from 0 to 1). It is usually acknowledged that a ROC curve the area under which has a value superior to 0.7 is a good predictive curve for diagnosis. The ROC curve has to be acknowledged as a curve allowing prediction of the quality of a diagnosis test.

<u>Results</u>

Data from a total of 111 patients were collected. Five patients were not eligible for the analysis: four with PFAPA syndrome and one with a diagnosis of CINCA with incomplete information.

A total of 106 patients were available for the analysis: 42 FMF, 39 CAPS, 14 TRAPS and 11 MKD.

Step 1 Physician's blinded web evaluation of patient's disease activity level:

During the three iterative web based blinded evaluation consensus was achieved on 63/106 cases (59.4%) with 13 patients declared as inactive, 49 active (26 with low activity, 13 moderate activity, 10 high activity) and one patient not evaluable (wrong diagnosis) by consensus.

For the remaining 43 patients consensus was not achieved and patients were therefore considered for the consensus NGT discussion as per Step 2.

Step 2 NGT consensus conference of patient's disease activity level

During the NGT meeting consensus was achieved for an additional 36 patients for a total of 98/106 cases (92%).

For the remaining eight cases consensus was not achieved and the patients were therefore discarded for further evaluation.

Table 2 reports the disease activity grading for the 4 HRFs diagnosed in 98 patients with consensus and for all patients combined. 26 out of 98 subjects (27%) were declared as inactive while the remaining 72 (63%) were classified as active (33 low, 28 moderate, 11 high disease activity).

Table 2: Consensus classification of the 98 patients with four HRFs according to the level of disease activity

|  | FMF N=39 | CAPS N=35 | TRAPS N=14 | MKD N=10 | TOTAL N=98 |
|---|---|---|---|---|---|
| Inactive | 8 | 12 | 6 | 0 | 26 |
| Active: |  |  |  |  |  |
|     Low activity | 13 | 14 | 2 | 4 | 33 |
|     Mild Activity | 11 | 8 | 3 | 6 | 28 |
|     Severe activity | 7 | 1 | 3 | 0 | 11 |

FMF: familial Mediterranean fever; MKD: mevalonate kinase deficiency; TRAPS: tumor necrosis factor receptor-associated periodic syndrome; CAPS; and cryopyrin-associated periodic syndromes

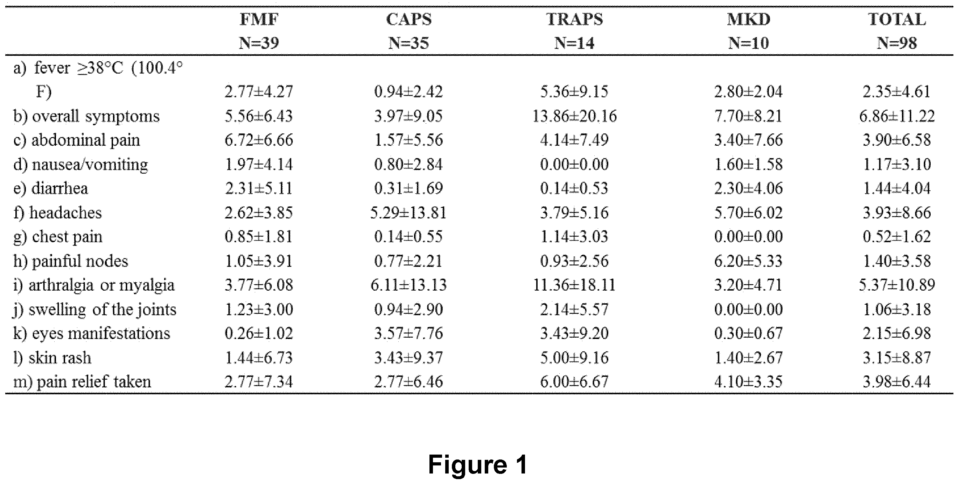

The descriptive statistics for each item of the AIDAI tool for the 98 patients is reported in Figure 1.

Step 3 Statistical analysis of the AIDAI scoring system.

Surprisingly, statistical analyses gave the same results when using the 0-3 score and the simplified no (0)/yes (1) version, only the latter is reported here.

In the final analytical step, in order to properly calculate sensitivity, specificity and ROC cut-off, the 98 patients were dichotomized as active (low, moderate and high activity combined) or inactive.

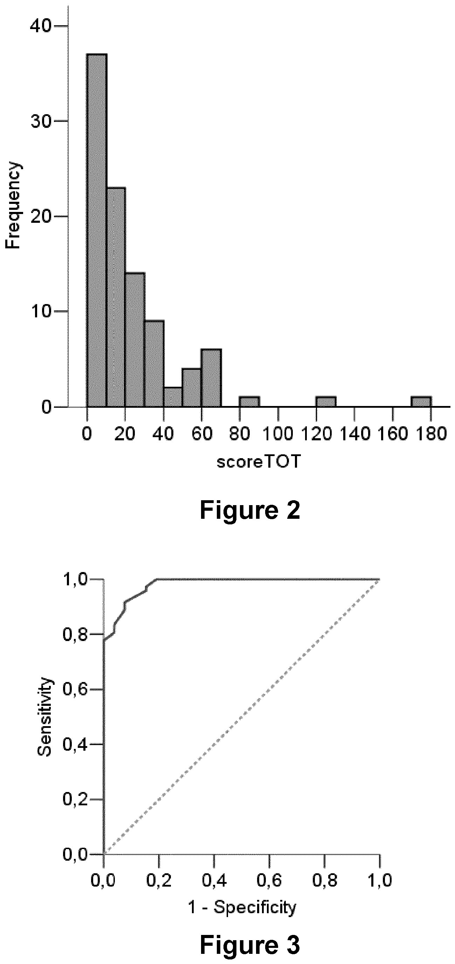

Figure 2 shows the distribution of the AIDAI total score with each AIDAI item dichotomized as yes/no: 14 subjects (12%) had a score=0; the score had a mean value of $22.2 \pm 26.8$) and a median of 14 (range = 0-175); values are skewed toward lower values of the AIDAI total score.

According to the ROC curve (Figure 3) an AIDAI score $\geq$ 9 points identifies active patients while an AIDAI total score < 9 points identifies patients as inactive; sensitivity was 89% (95%CI=80%-94%), specificity 92% (95% CI=76%-98%), area under the curve (AUC) of 98% (95% CI=96%-100%) and accuracy 90% (95% CI=84%-96%) (Table 3).

Table 3. Accuracy measures of the AIDAI total score (0/1 scoring) for 98 patients with a HRF diagnosis. A total AIDAI score <9 separates inactive from active patients (total score $\geq$9).

| Activity as defined by the AIDAI total score | Activity as defined by consensus | | TOTAL |
|---|---|---|---|
|  | Active | Inactive |  |
| $\geq$9 active | 64 (89%) | 2 (8%) | 66 |
| <9 inactive | 8 (11%) | 24 (92%) | 32 |
| TOTAL | 72 | 26 | 98 |

Similar performances were obtained when the original 0-3 score for each item of the AIDAI was applied as follows: sensitivity was 92% (95% CI=86%-98%), specificity 96% (95% CI=88%-100%), area under the curve (AUC) of 99% (95% CI=97%-100%) and accuracy 93% (95% CI=87%-98%) (cut-off at 10, data not shown).

Accurate and reproducible evaluation of disease activity is of major importance in the assessment of treatment efficacy, in appreciation of the effect of disease activity on quality of life, and, although this is currently unproven, may predict the development of serious long-term complications.

AIDAI is the first validated instrument designed to standardize assessment of AID activity across trials, and to facilitate comparison and meta-analysis of clinical trials in the future. AIDAI will also allow patient's self-reported disease activity evaluation in daily practice.

AIDAI proved to be a valid and reliable tool for the assessment of presence or absence of disease activity in the four main HRFs.

In its current form, the AIDAI score is very easy to use. A unique patient/parents diary gathering all variables for the four main auto-inflammatory diseases is convenient for routine clinical use. The statistical analyses showed that a simple sum of the positive items from the diary with binary value (yes/no) has the same statistical performance as a disease-specific scoring system with graded values reported in the prior art (Piram *et al.*, *op. cit.*).

Both patients and physicians will benefit from this simplified scoring system, as binary values are easier for patients and the single format with a straightforward sum of all positive items simplifies its use by physicians.

In this validation phase, patients were asked to complete a one month prospective diary prior to their scheduled clinical appointment. However the diary could be completed for periods of other lengths as clinically appropriate. A three month period survey is probably more suitable for episodic diseases such as FMF and MKD but the period could be longer in TRAPS and shorter in CAPS.

For use in a period greater than one month, the calculation of the score is straightforward consisting of the sum of all 12 variables as indicated above, divided by the number of months over which the diary was completed.

An AIDAI cut-off score of nine accurately differentiated patients with active versus inactive disease with an area under the ROC curve of 0.98 with sensitivity and specificity exceeding 0.8. This cut-off will help physicians in daily practice to decide if a patient has active or inactive disease. This is moreover important for clinical trials in which an index discriminating patients achieving remission from those who did not, might increase the power of the statistical comparisons, thus permitting smaller sample size (Turner et al., Gastroenterology 2007;133(2):423-32) a crucial factor in orphan and ultra-orphan diseases such as HRFs.

Although the number of patients followed made is possible to design the AIDAI score, the limited number of patients did not allow for separate analyses of the different diseases or discrimination with sufficient power of the different levels of disease activity (low, moderate and high).

The AIDAI score was validated for the four main AIDs, but could also be applied to other AID which shares some of their clinical features such as PFAPA (Periodic Fever Aphtous stomatitis Pharyngitis Adenitis) and Schnitzler's syndrome.

Furthermore, once could develop disease-specific scores by not taking into account the variables that are not associated with some diseases. In particular, on could sum the following variables for a period of one month:

FMF: fever ≥38°C / abdominal pain / chest pain / arthralgia or myalgia / swelling of the joints / skin rash
MKD: fever ≥38°C / abdominal pain / nausea/vomiting / diarrhea / painful nodes / arthralgia or myalgia
TRAPS: fever ≥38°C / overall symptoms / abdominal pain / arthralgia or myalgia / eyes manifestations / skin rash
CAPS: fever ≥38°C / headaches / arthralgia or myalgia / eyes manifestations / skin rash

The threshold for determining activity for these diseases should also be close to 9 and could be easily determined according to the method as described above.

This tool can be useful in clinical practice as well as clinical trials, in the assessment of activity of these rare diseases, thus permitting more reliable analysis of the efficacy of new treatments.

**Claims**

1. A computer-implemented *ex vivo* method for determining the activity of an auto-inflammatory disease in a patient, wherein said auto-inflammatory disease is chosen in the group consisting of a hereditary recurrent fever syndrome (HRFs), mevalonate kinase deficiency (MKD), tumor necrosis factor receptor (TNF)-associated periodic syndrome (TRAPS), and cryopyrin-associated periodic syndromes (CAPS), comprising the steps of:

a) Having said patient daily report, during a one-month period, the status of 12 items consisting of Fever ≥38°C, Overall symptoms, Abdominal pain, Nausea/vomiting, Diarrhea, Headaches, Chest pain, Painful nodes, Arthralgia or Myalgia, Swelling of the joints, Eyes manifestations, Skin rash,

b) Recovering said reports from said patient

c) Allocating the value "1" to each item which was noted as being present by said patient and the value "0" to each item which was noted as being absent by said patient

d) Combining, through an algebraic sum, all the values obtained in step c) for each days of a consecutive period of one month in order to obtain an end value,

wherein said auto-inflammatory disease is active in said patient if said end-value is higher than a predetermined cut-off, wherein said predetermined cut-off is 9.

2. The method of claim 1, further comprising the step of providing a one-month diary to said patient, wherein said diary comprises an emplacement for each day of the month and wherein each daily emplacement of said diary comprises a list of 12 items consisting of Fever ≥38°C, Overall symptoms, Abdominal pain, Nausea/vomiting, Diarrhea, Headaches, Chest pain, Painful nodes, Arthralgia or Myalgia, Swelling of the joints, Eyes manifestations, Skin rash, before step a).

3. The method of claim 1, wherein said auto-inflammatory disease is a major hereditary recurrent fever syndrome.

4. The method of any of claims 1 or 3, wherein said report in step a) is performed by said patient filling an electronic form on a patient's device.

5. The method of claim 4, wherein said data entered by the patient on step a) is sent to a distant server after completion and validation.

6. The method of claim 5, wherein said communication between said distant server and said patient's device is encrypted.

7. The method of any of claims 4 to 6, wherein said patient is automatically reminded every day to fill the electronic form.

8. The method of any of claims 4 to 7, wherein said allocation of the 0/1 value of step c) and said calculation of the end value (calculated on a one month rolling) in step d) are performed automatically on the distant server, and said end value is sent to the physician/clinician in charge of said patient.

**Patentansprüche**

1. Computerimplementiertes ex vivo-Verfahren zum Bestimmen der Aktivität einer autoinflammatorischen Erkrankung bei einem Patienten, wobei die autoinflammatorische Erkrankung ausgewählt ist aus der Gruppe bestehend aus einem hereditären rekurrierenden Fiebersyndrom (HRFs), Mevalonatkinase-Defizienz (MKD), Tumornekrosefaktor-Rezeptor- (TNF)-assoziiertem periodischem Symdrom (TRAPS) und cryopyrin-assoziierten periodischen Syndromen (CAPS), umfassend die Schritte:

a) tägliches Bericherstattendes Patient während eines Zeitraums von einem Monat über den Status von 12 Elementen berichtet, die aus Fieber ≥ 38 °C, Gesamtsymptomen, Bauchschmerzen, Übelkeit/Erbrechen, Diarrhoe, Kopfschmerzen, Brustschmerzen, schmerzhaften Knotenpunkten, Arthralgie oder Myalgie, Schwellung der Gelenke, Augenmanifestationen, Hautausschlag,

b) Erhalten der Berichte von dem Patienten,

c) Zuweisen des Werts von "1" zu jedem Element, der von dem Patienten als vorhanden notiert wurde, und des Werts von "0" zu jedem Element, der von dem Patient als fehlend notiert wurde,

d) Kombinieren aller Werte, die in Schritt c) erhaltenen wurden, über eine algebraische Summe für jeden Tag eines aufeinander folgenden Zeitraums von einem Monat, um einen Endwert zu erhalten,

wobei die autoinflammatorische Erkrankung in dem Patienten aktiv ist, falls der Endwert höher als ein vorbestimmter Grenzwert ist, wobei der vorbestimmte Grenzwert 9 ist.

2. Verfahren nach Anspruch 1, ferner umfassend vor Schritt a) den Schritt des Bereitstellen eines einmonatigen Tagebuchs an den Patienten, wobei das Tagebuch eine Eintragfläche für jeden Tag des Monats umfasst, und wobei jede tägliche Eintragfläche des Tagebuchs eine Liste von 12 Punkten umfasst, die aus Fieber ≥ 38 °C, Gesamtsymptomen, Bauchschmerzen, Übelkeit/Erbrechen, Diarrhoe, Kopfschmerzen, Brustschmerzen, schmerzhaften Knotenpunkten, Arthralgie oder Myalgie, Schwellung der Gelenke, Augenmanifestationen, Hautausschlag bestehen.

3. Verfahren nach Anspruch 1, wobei die autoinflammatorische Erkrankung ein hereditäres rekurrierendes Fiebersyndrom der Major-Form ist.

4. Verfahren nach einem der Ansprüche 1 oder 3, wobei der Bericht in Schritt a) durch den Patienten durchgeführt wird, der ein elektronisches Formular auf einem Patientengerät ausfüllt.

5. Verfahren nach Anspruch 4, wobei die Daten, die von dem Patienten in Schritt a) eingegebenen werden, nach vollständigem Ausfüllen und Validierung zu einem entfernten Server gesendet werden.

6. Verfahren nach Anspruch 5, wobei die Kommunikation zwischen dem entfernten Server und dem Patientengerät verschlüsselt wird.

7. Verfahren nach einem der Ansprüche 4 bis 6, wobei der Patient automatisch jeden Tag daran erinnert wird, das elektronische Formular auszufüllen.

8. Verfahren nach einem der Ansprüche 4 bis 7, wobei das Zuweisen des 0/1-Wertes in Schritt c) und das Berechnen des Endwerts (gleitend berechnet für einen Monat) in Schritt d) automatisch auf dem entfernten Server durchgeführt werden und der Endwert an den Arzt/Kliniker gesendet wird, der für den Patienten zuständig ist.


**Revendications**

1. Procédé *ex vivo* mis en oeuvre par ordinateur pour déterminer l'activité d'une maladie auto-inflammatoire chez un patient, ladite maladie auto-inflammatoire étant choisie dans le groupe constitué par un syndrome de fièvre récurrente héréditaire (HRFs), le déficit en mévalonate kinase (MKD), le syndrome périodique associé au récepteur du facteur de nécrose tumorale (TNF) (TRAPS), et les syndromes périodiques associés à la cryopyrine (CAPS), comprenant les étapes consistant à :

a) faire en sorte que ledit patient rapporte quotidiennement, pendant une période d'un mois, l'état de 12 items constitués par une fièvre $\geq$ 38 °C, des symptômes globaux, une douleur abdominale, des nausées/ vomissements, une diarrhée, des maux de tête, une douleur thoracique, des nodules douloureux, une arthralgie ou une myalgie, un gonflement des articulations, des manifestations oculaires, une éruption cutanée,
b) récupérer lesdits rapports auprès dudit patient,
c) affecter la valeur « 1 » à chaque item qui a été noté comme étant présent par ledit patient et la valeur « 0 » à chaque item qui a été noté comme étant absent par ledit patient,
d) combiner, par une somme algébrique, toutes les valeurs obtenues à l'étape c) pour chaque jour d'une période consécutive d'un mois afin d'obtenir une valeur finale,

dans lequel ladite maladie auto-inflammatoire est active chez ledit patient si ladite valeur finale est supérieure à une limite prédéterminée, ladite limite prédéterminée étant de 9.

2. Procédé selon la revendication 1, comprenant en outre l'étape consistant à fournir un journal d'un mois audit patient, ledit journal comprenant un emplacement pour chaque jour du mois et chaque emplacement quotidien dudit journal comprenant une liste de 12 items constituée par une fièvre $\geq$ 38 °C, des symptômes globaux, une douleur abdominale, des nausées/vomissements, une diarrhée, des maux de tête, une douleur thoracique, des nodules douloureux, une arthralgie ou une myalgie, un gonflement des articulations, des manifestations oculaires, une éruption cutanée, avant l'étape a).

3. Procédé selon la revendication 1, dans lequel ladite maladie auto-inflammatoire est un syndrome majeur de fièvre récurrente héréditaire.

4. Procédé selon l'une quelconque des revendications 1 et 3, dans lequel ledit rapport à l'étape a) est effectué par ledit patient remplissant un formulaire électronique sur un dispositif de patient.

5. Procédé selon la revendication 4, dans lequel lesdites données entrées par le patient à l'étape a) sont envoyées à un serveur distant après achèvement et validation.

6. Procédé selon la revendication 5, dans lequel la communication entre ledit serveur distant et ledit dispositif de patient est chiffrée.

7. Procédé selon l'une quelconque des revendications 4 à 6, dans lequel ledit patient se voit rappeler automatiquement tous les jours de remplir le formulaire électronique.

8. Procédé selon l'une quelconque des revendications 4 à 7, dans lequel ladite affectation de la valeur 0/1 de l'étape c) et ledit calcul de la valeur finale (calculée sur un roulement d'un mois) à l'étape d) sont effectués automatiquement sur le serveur distant, et ladite valeur finale est envoyée au médecin/clinicien en charge dudit patient.

| | FMF N=39 | CAPS N=35 | TRAPS N=14 | MKD N=10 | TOTAL N=98 |
|---|---|---|---|---|---|
| a) fever ≥38°C (100.4° F) | 2.77±4.27 | 0.94±2.42 | 5.36±9.15 | 2.80±2.04 | 2.35±4.61 |
| b) overall symptoms | 5.56±6.43 | 3.97±9.05 | 13.86±20.16 | 7.70±8.21 | 6.86±11.22 |
| c) abdominal pain | 6.72±6.66 | 1.57±5.56 | 4.14±7.49 | 3.40±7.66 | 3.90±6.58 |
| d) nausea/vomiting | 1.97±4.14 | 0.80±2.84 | 0.00±0.00 | 1.60±1.58 | 1.17±3.10 |
| e) diarrhea | 2.31±5.11 | 0.31±1.69 | 0.14±0.53 | 2.30±4.06 | 1.44±4.04 |
| f) headaches | 2.62±3.85 | 5.29±13.81 | 3.79±5.16 | 5.70±6.02 | 3.93±8.66 |
| g) chest pain | 0.85±1.81 | 0.14±0.55 | 1.14±3.03 | 0.00±0.00 | 0.52±1.62 |
| h) painful nodes | 1.05±3.91 | 0.77±2.21 | 0.93±2.56 | 6.20±5.33 | 1.40±3.58 |
| i) arthralgia or myalgia | 3.77±6.08 | 6.11±13.13 | 11.36±18.11 | 3.20±4.71 | 5.37±10.89 |
| j) swelling of the joints | 1.23±3.00 | 0.94±2.90 | 2.14±5.57 | 0.00±0.00 | 1.06±3.18 |
| k) eyes manifestations | 0.26±1.02 | 3.57±7.76 | 3.43±9.20 | 0.30±0.67 | 2.15±6.98 |
| l) skin rash | 1.44±6.73 | 3.43±9.37 | 5.00±9.16 | 1.40±2.67 | 3.15±8.87 |
| m) pain relief taken | 2.77±7.34 | 2.77±6.46 | 6.00±6.67 | 4.10±3.35 | 3.98±6.44 |

**Figure 1**

EP 2 962 233 B1

**Figure 2**

**Figure 3**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **TOUITOU ; KONE-PAUT.** Autoinflammatory diseases. *Best Pract Res Clin Rheumatol,* 2008, vol. 22 (5), 811-29
- **HASHKES et al.** *Ann Intern Med,* 2012, vol. 157 (8), 533-41
- **SINGH et al.** *Arthritis Rheum,* 2006, vol. 55 (3), 348-52
- **HOFFMAN et al.** *Arthritis Rheum,* 2008, vol. 58 (8), 2443-52
- **LACHMANN et al.** *N Engl J Med,* 2009, vol. 360 (23), 2416-25
- **GILLESPIE et al.** *J Inflamm Res,* 2010, vol. 3, 1-8
- **PIRAM et al.** *Ann Rheum Dis,* 2011, vol. 70 (2), 309-14
- **GRATEAU.** *Acta Clin Belg.,* September 2006, vol. 61 (5), 264-9
- **FILOCAMO et al.** *J Rheumatol,* 2010, vol. 37 (7), 1534-41
- **PINCUS et al.** *J Rheumatol,* 2008, vol. 35 (8), 1550-8
- **TURNER et al.** *Gastroenterology,* 2007, vol. 133 (2), 423-32